(19) Europäisches Patentamt
European Patent Office
Office européen des brevets

(11) Publication number: **0 021 750**
**B1**

(12) **EUROPEAN PATENT SPECIFICATION**

(45) Date of publication of patent specification: **26.09.84**

(51) Int. Cl.$^3$: **C 08 B 37/08**

(21) Application number: **80302001.5**

(22) Date of filing: **13.06.80**

(54) **Production of spherically shaped material made of chitin derivative.**

(30) Priority: **15.06.79 JP 75401/79**

(43) Date of publication of application:
**07.01.81 Bulletin 81/01**

(45) Publication of the grant of the patent:
**26.09.84 Bulletin 84/39**

(84) Designated Contracting States:
**DE FR GB IT NL SE**

(56) References cited:
**EP-A-0 013 181**
**DE-C- 894 993**
**US-A-4 111 810**

**PROCEEDINGS INTERNATIONAL CONFERENCE CHITIN/CHITOSAN, 1st 1977 published May 1978 G.K. MOORE et al.: "Studies on the acetylation of chitosan", pages 421-429**

(73) Proprietor: **KUREHA KAGAKU KOGYO KABUSHIKI KAISHA**
**9-11 Horidome-cho 1-chome Nihonbashi Chuo-ku**
**Tokyo (JP)**

(72) Inventor: **Koshugi, Junichi**
**No. 22 Daiichi-Kurehasoh 3-10-13 Nerima**
**Nerima-Ku**
**Tokyo (JP)**

(74) Representative: **Myerscough, Philip Boyd et al**
**J.A.Kemp & Co. 14, South Square Gray's Inn**
**London, WC1R 5EU (GB)**

**EP 0 021 750 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for producing spherically shaped materials and to their use.

Fibre-forming polysacchrides occurring in nature are divided broadly into collagen in higher animals, chitin in arthropods and lower plants and cellulose in higher plants. The skeletons of the living things are made by sedimentation of apatite, calcium carbonate and lignin respectively onto these polysaccharides.

Chitin is a mucopolysaccharide of poly-N-acetyl-D-glucosamine. The amount of it in nature compares favorably with the amount of cellulose. However, since chitin is a highly crystalline substance and its inter-molecular bonding through the aminoacetyl group is extremely stable, it is very difficult to find an appropriate solvent to dissolve, disperse or swell the chitin satisfactorily. Accordingly, development of the use of chitin resources is far behind that of cellulose and other polysaccharides.

DE-C-894993 describes the acylation of de-N-acetylated chitin (chitosan). Acylation is a solid-liquid reaction in which solid de-N-acetylated chitin is reacted with, for example to regenerate chitin, acetic acid/methanol or glacial acetic acid.

According to the recent research, films or fibres consisting of regenerated chitin can be obtained by the acetylation of a chitosan by utilizing the properties of chitosan dissolved in an aqueous dilute acid solution. For instance, Japanese Patent Publication No. 19213/73 discloses a method for obtaining a film of a regenerated chitin by making a film of chitosan followed by acetylating the thus-formed film in the solid phase. The method comprises a series of steps. First, the chitosan is dissolved in an aqueous dilute acid solution. A film of a chitosan-salt obtained by evaporating off water is immersed in an aqueous dilute alkali solution or a water-containing solution of an organic base in a solvent therefor to prepare the film of chitosan. After swelling the thus obtained film of chitosan in water, the swollen film is soaked in a solvent such as ethanol, acetone or pyridine and is N-acetylated with acetic acid in the presence of dicyclohexylcarbodiimide as a dehydrating condensation agent to obtain a film of regenerated chitin. However, it takes a long time to complete the acetylation. Moreover, the object of the published reference is to obtain films of regenerated chitin. The only use suggested for the regenerated chitin is as an acoustic vibrator plate. Accordingly, this method is still unsatisfactory for the view point of utilization of chitin resources.

It has now been discovered that spherically shaped materials which have at least an outer layer of an acylated de-N-acetylated chitin can be prepared from a solution of a de-N-acetylated chitin or a salt thereof and a liquid containing an organic acid anhydride, as an acylating agent, and a suspending agent. This technique enables chitin derivatives to be utilised in a wide variety of applications. The materials may consist of the acylated de-N-acetylated chitin throughout or their interior may comprise the original de-N-acetylated chitin from which the acylated product has been formed.

Accordingly, the present invention provides a process for producing a spherically shaped material having an outer surface layer which comprises an acylated de-N-acetylated chitin or a salt thereof, which process comprises contacting an aqueous solution of a de-N-acetylated chitin or a salt thereof with a liquid containing an organic acid anhydride and a suspending agent in such a manner as to produce the material in the desired shape.

As mentioned previously, the interior of the shaped materials may comprise a de-N-acetylated chitin or a salt thereof, or it may consist entirely or almost entirely of the acylated de-N-acetylated chitin or salt thereof, in which case the shaped material will wholly or substantially wholly be made up of this acylated product.

The surface layer of acylated de-N-acetylated chitin of the shaped materials is insoluble in water, acids, alkaline solutions and organic solvents.

It is chemically and physiologically stable and safe, and can exhibit excellent permeability, adsorbability and bio-compatibility.

The spherically shaped materials can be employed as an adsorbent, for example for use in a chromatography column or in an ion-exchanger, as a base material on which an enzyme, antibody or antigen is immobilized or as capsules, for example for slow-releasing medicines. They can be used in dialysis, for example in catching heavy metals, or in ultrafiltration.

The acylated de-N-acetylated chitin of the surface layer of the shaped materials may be obtained by acylating a de-N-acetylated chitin which can be represented by the following averaged general formula:

$$[C_6H_9O_4\cdot(NH_2)_x\cdot(NHCOCH_3)_y]_n \qquad (I)$$

wherein x is from 0.5 to 1.0 and y = 1.0 − x, or is a salt thereof. This de-N-acetylated chitin may in turn be obtained by de-N-acetylating chitin.

Chitin is a naturally produced mucopolysaccharide with the following formula:

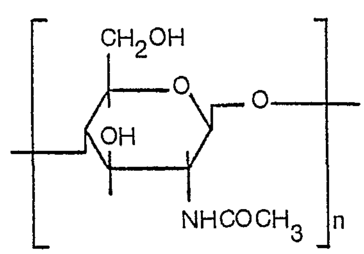

(II)

wherein *n* denotes the degree of polymerization.

Although *n* in the formula (II) can not be determined accurately because no good solvent for suitably dissolving or dispersing chitin exists at present, *n* is believed to be commonly in the range of from 50 to 10,000.

De-N-acetylation of chitin is achieved by heating the chitin in a concentrated aqueous solution of an alkali such as sodium hydroxide or potassium hydroxide. The de-N-acetylated chitin usually has a degree of de-N-acetylation, on average, of from 0.5 to 1.0 per pyranose ring, preferably 0.5 to 0.9.

The de-N-acetylated chitin is soluble in an aqueous dilute acid solution. The solution may be one of an organic acid, such as acetic acid or oxalic acid, or of an inorganic acid, such as hydrochloric acid, which has a concentration sufficient to dissolve the de-N-acetylated chitin. In addition salts of de-N-acetylated chitin are soluble in water. The salt can be an acetate or a hydrochloride.

Shaped materials are produced by adding an aqueous solution of the de-N-acetylated chitin to an anhydride of an organic acid or a solution thereof containing a suspending agent. The anhydride acts as an acylating agent. If necessary, the liquid containing the organic acid anhydride also contains an organic acid. Depending on the manner in which the shaped materials are formed, the aqueous solution of the de-N-acetylated chitin may need to be dispersed in the organic acid anhydride or solution thereof.

The concentration of the de-N-acetylated material in aqueous solution affects the strength and the density of the shaped materials that are produced. When it is higher, the shaped materials of larger strength and a denser structure are obtained. Such shaped materials act as a molecular sieve over a broad range of molecular diameters. Usually the concentration of the de-N-acetylated chitin in the aqueous solution is 0.1 to 10% by weight. However, the concentration should be adequately adjusted according to the intended use and desired properties of the shaped materials and it is not necessarily restricted to this range.

When the viscosity of the aqueous solution of the de-N-acetylated chitin is high, sometimes it is difficult to obtain spherical particles with a small diameter. In such a case, a viscosity-reducing agent, such as ethylene glycol, glycerol or alcohol, may be added to the aqueous solution of the de-N-acetylated chitin.

In the process of the invention, the anhydride or an organic acid and the organic acid are preferably aliphatic or aromatic acids with from one to twenty carbon atoms and their anhydrides, for example acetic acid, propionic acid, butyric acid, valeric acid and benzoic acid and their anhydrides. The anhydride may be used singly or a mixture or more than one may be employed. Although the amount of acylating agent is not particularly restricted, it is usually from 1 to 100 times by equivalent, preferably from 5 to 20 times by equivalent, per equivalent of amino groups of the de-N-acetylated chitin.

The organic acid anhydride may be used as it is without dilution, or may be diluted with the organic acid or diluted with or dissolved in an organic solvent which does not react with the anhydride, for instance benzene, toluene, xylene or decalin. Such dilution can enable the reaction velocity to be controlled or facilitate the treatment of the reaction product. In addition, in order to facilitate the control of the state of dispersed particles of de-N-acetylated chitin in the solution of the organic acid anhydride, it is preferable to add the organic solvent to the reaction system in an amount of from 10 to 1,000 times by weight, preferably from 10 to 500 times by weight of the de-N-acetylated chitin.

The suspending agent used in the process is preferably non-ionic, for instance a polyoxyethylene sorbitan monoester or a sorbitan monoester such as polyoxyethylene sorbitan monooleate, polyoxyethylene sorbitan monostearate, polyoxyethylene sorbitan monopalmitate, polyoxyethylene sorbitan monolaurate, sorbitan monooleate, sorbitan monostearate, sorbitan monopalmitate or sorbitan monolaurate. The amount of suspending agent may be selected as desired in the range of from 0.001 to 10% by weight of the amount of the aqueous solution of the de-N-acetylated chitin. The suspending agent may be incorporated in the aqueous solution of the de-N-acetylated chitin as well in the solution of the anhydride.

The temperature of the acylation may be from 5 to 80°C, preferably from 5 to 60°C.

Although the acylation mechanism has not been elucidated, reaction takes place as soon as the aqueous solution of de-N-acetylated chitin is brought into contact with the organic acid anhydride. The reaction proceeds from the surface of the aqueous de-N-acetylated chitin solution to be formed into a shaped material. An insoluble membrane of the acylated product is formed on the surface of the aqueous de-N-acetylated chitin solution. Inside this membrane the solution is still an aqueous de-N-acetylated chitin solution. As the reaction proceeds, the organic acid anhydride diffuses into the interior across the membrane to cause acylation therein gradually. This results in insolubilization of the interior. Accordingly, it is possible to produce a spherically shaped material having a surface layer comprising the insoluble acylated product whilst its interior comprises unreacted de-N-acetylated chitin or a salt thereof, depending on the extent to which acylation has proceeded. Acylation predominantly takes place on the N-atom which is bonded to the 2-carbon atom of the pyranose ring of the de-N-acetylated chitin, as shown, for example, by the following formulae (III) to (V). Acylation may also occur on one or both of the O-atoms bonded to the 3-and 5-carbon atoms of the pyranose ring as in the following formulae (IV) and (V):

$$C_6H_8O_3\cdot(OH)\cdot NHCOR' \quad (III)$$

$$C_6H_8O_3\cdot(OCOR')\cdot NHCOR' \quad (IV)$$

$$C_6H_7O_2\cdot(OCOR')_2\cdot NHCOR' \quad (V)$$

wherein R' is an alkyl group or an aryl group.

Acylation may be accomplished by pouring, preferably dropwise, the aqueous solution of de-N-acetylated chitin into the liquid containing the anhydride and the suspending agent, or *vice versa*, and then vigorously and homogeneously dispersing the aqueous solution of the de-N-acetylated chitin within the acylating solution by using, for example, a stirrer of the blade type, a static mixer or a homogenizer. In this manner, reaction takes place at once from the surface of the drop of the aqueous solution of the de-N-acetylated chitin to form an insolubilized membrane of the acylated product. In addition, by spraying an aqueous solution of de-N-acetylated chitin into a mist of the acid anhydride, ultramicrospherical particles may be obtained, the surface layer of the ultramicrospherical droplets being infusibilized instantly by acylation.

The size of spherical particles of the invention can be adjusted within a broad range of diameters from $10^{-7}$ to $5\times10^{-3}$ m (0.1 to 5,000 $\mu$m). The degree of sphericity of the particles is such that the maximum diameter of the particles is about 1.0 to 1.3 times their minimum diameter in the wet state.

The thickness and compactness of the insolubilized surface layer of shaped materials are variable. These properties can be controlled depending on the concentration of the aqueous solution of the de-N-acetylated chitin, the time and temperature of the acylation reaction. The thickness may be from $10^{-7}$ to $10^{-3}$ m (0.1 to 1,000 $\mu$m) and the compactness such that the materials have a permeability threshold of from 500 to 100,000 in molecular weight.

The shaped materials of the present invention may be subjected to a cross-linking treatment, if necessary. One such treatment is as follows:

First, the moisture content of the material is adjusted to twice to 3 times the weight of the dried material. Then the material is soaked in an aqueous sodium hydroxide solution at a concentration more than 40% by weight and in an amount of more than twice, preferably from 4 to 30 times by weight of the dried material, at a temperature lower than 15°C for 1 to 5 hours. After that, excess sodium hydroxide solution is removed. The material is left to stand soaked in the aqueous sodium hydroxide solution in an amount of from 3 to 6 times the weight of the dried material at a temperature of from 0 to 10°C for 1 to 24 hours to obtain a material pretreated with alkali. After freeze-drying the pretreated material at a temperature of 0 to −30°C for 1 to 24 hours (an optional step), the pretreated material is dispersed in an aqueous solution or an organic solvent containing a cross-linking agent in an amount of from 0.1 to 3 molar times, preferably 0.5 to 2 molar times, the molar amount of pyanose rings and is reacted at a temperature lower than 15°C for from 5 to 48 hours. After the reaction is over, the reaction product is washed and neutralized to obtain the cross-linked material. As the cross-linking agent, epoxy agents such as epichlorohydrin, epibromopropanol and 2,3-dichloro-propanol are suitable. The degree of cross-linking may be sufficient at up to 0.3 per pyranose ring.

In addition, when the interior of the shaped material is not acylated or insolubilized, the cross-linking agent may be selected from aldehydes, such as formaldehyde, glyoxal and glutaraldehyde, which from a Schiff base with the de-N-acetylated chitin in the interior of the material.

The shaped materials are composed of the chemically and biologically stable and safe acylated product and are permeable. Consequently, they are applicable over a broad range of uses. The spherical particles may be used in the separation and purification of polysaccharides, proteins, and the mixtures thereof, by immersing the materials in an aqueous solution of these substances of various molecular weights. Only substances of a molecular weight less than a predetermined value are dispersed into the materials. Moreover, when the materials have amino groups in their structures, they can also be utilized in ion-exchangers. Because of the stability and safety of the materials in relation to living bodies, they can be used for instance, for blood perfusion, for pads for surgical injuries, as an absorbent of gastrointestinal toxins or to coat an adsorbent.

In addition, where the materials are used in contact with blood, it is preferable to adjust the acylation to leave some free amino groups and then to make a polyion with an anti-thromobolytic agent such as heparin, chitosan sulfate or chitin sulfate.

Also, it is possible to confine several useful substances within the spherical particles of the present invention. For instance, by encapsulating one or several ion-exchange resins within the particles, an ion-exchange body coated by a chitinic membrane is obtained which has ion-exchange activity limited to substances with a molecular weight less than a predetermined value. By coating an adsorbent, such as activated carbon, with the material of the present invention, an adsorbent having a functional ability never known before is obtained.

The following Examples illustrate the present invention.

Example 1

A solution of $5 \times 10^{-3}$ kg (5 g) of de-N-acetylated chitin with a degree of de-N-acetylation of 0.8 dissolved in $2 \times 10^{-4}$ $m^3$ (200 ml) of an aqueous 2% acetic acid solution was divided into two equal portions. Into one of the portions, 1% by weight of sorbitan monooleate and 20% by weight of ethanol were added. Into the other portion, 20% by weight of ethanol was added. The former portion was designated *A* liquid. The latter portion was designated *B* liquid. Separately, two sets of dispersing media were prepared by dissolving in each $5 \times 10^{-3}$ kg (5 g) of polyoxyethylene sorbitan monooleate and $10^{-2}$ kg (10 g) of acetic anhydride as an acylating agent, in $2 \times 10^{-3}$ $m^3$ (2 liters) of toluene in a dispersion vessel provided with a bow-type stirrer. Then, *A* liquid and *B* liquid were added to respective dispersing media while stirring at 1,000 r.p.m. at room temperature for one hour to acylate the *A* and *B* liquids. On adding $10^{-3}$ $m^3$ (one liter) of ethanol to the respective reaction mixtures while agitating, clear spherical particles were formed and precipitated. After collecting the particles by filtration and washing them with ethanol, they were dispersed in $10^{-3}$ $m^3$ (one liter) of distilled water. The pH of the water was adjusted to 8.0. After collecting the thus-treated particles by filtration and washing off sodium acetate and sodium hydroxide adhered to the particles with distilled water, gel-like spherical particles of the present invention were obtained having a diameter of $5 \times 10^{-5}$ to $1.5 \times 10^{-4}$ m (50 to 150 microns).

The gel-like particles prepared from both liquids *A* and *B* were insoluble in an aqueous 5% by weight solution of acetic acid, and are elastic, containing water. After drying, both the particles prepared from liquid *A* and from liquid *B* did not show an infrared absorption band of 1,500 to 1,530 $cm^{-1}$ due to amino group, which showed they were completely acylated. Their elementary analyses gave the following values:

| Product | C | H | O | N (%) |
|---|---|---|---|---|
| from *A* | 46.5 | 6.4 | 40.4 | 6.8 |
| from *B* | 46.3 | 6.5 | 40.3 | 6.7 |

These values show fairly good coincidence with those of chitin.

Example 2

Two columns each with an inner diameter of $2 \times 10^{-2}$ m (2 cm) were filled respectively with particles *A* (prepared from liquid *A* of Example 1 containing a surfactant) and particles *B* (prepared from liquid *B* of Example 1 not containing a surfactant) up to a height corresponding to a volume of $1.5 \times 10^{-4}$ $m^3$ (150 ml) in the column. The columns were tested to assess the separating ability of particles *A* and *B* as follows:

$2 \times 10^{-5}$ kg (20 mg) of each of blue-dextran of molecular weight of 2,000,000, of dextran (i) of molecular weight 100,000, of dextran (ii) of molecular weight 10,000 and of glucose were dissolved in $2 \times 10^{-6}$ $m^3$ (2 ml) of distilled water. The solution was developed on a column. Distilled water was poured onto the column at a rate of $1.66 \times 10^{-8}$ $m^3/s$ (1 ml/min). The effluent was collected every $5 \times 10^{-6}$ $m^3$ (5 ml) in portions to analyze the solutes therein. The results of analyses are shown in Table 1.

As is seen in Table 1, the particles according to the present invention separated the mixture of blue dextran, the two dextrans (i) and (ii) and glucose into the individual components thus showing the effectiveness of the particles as a column-filling material for chromatography. In particular particles *A* are noticeable in their ability to separate dextran of molecular weight 100,000.

TABLE 1
Results of Chromatographic Separation

| | Position of the solute in the effluent shown by the volume of the effluent ($m^3 \times 10^{-6}$) | |
|---|---|---|
| Solute | Shaped material *A* | Shaped material *B* |
| Blue-dextran | 55 to 65 | 55 to 65 |
| Dextran (i) | 80 to 90 | 55 to 65 |
| Dextran (ii) | 90 to 100 | 90 to 100 |
| Glucose | 150 to 160 | 155 to 165 |

## Example 3

Particles in accordance with the invention were prepared by acylating *A* liquid of Example 1 with propionic anhydride as in the manner of Example 1. The reaction time was lengthened to three hours.

The particles were acylated completely throughout, and did not dissolve in an aqueous 5% by weight of acetic acid solution but maintained their spherical shape. The size of the particles was in the range of from $5 \times 10^{-5}$ to $1.5 \times 10^{-4}$ m (50 to 150 $\mu$m). The ratio of the maximum diameter to the minimum diameter of the particles was in the range of from about 1.0:1 to 1.1:1.

## Example 4

$10^{-3}$ kg (one gram) of de-N-acetylated chitin with a degree of de-N-acetylation of 0.9 was dissolved in $10^{-4}$ m³ (100 ml) of an aqueous 2% by weight of acetic acid solution. $10^{-4}$ m³ (10 ml) of methanol was added to the solution to obtain a homogeneous solution. Separately, $2 \times 10^{-2}$ kg (20 g) of polyoxyethylene sorbitan monooleate and $5 \times 10^{-3}$ kg (5 g) of acetic anhydride were dissolved in $3 \times 10^{-3}$ m³ (3 liters) of toluene in a dispersing vessel provided with a bow-type stirrer. The solution of de-N-acetylated chitin was added and acylated in the dispersing vessel while stirring at 10,000 r.p.m. at room temperature for two hours. After adding $2 \times 10^{-3}$ m³ (2 liters) of ethanol, a precipitate separated out. After separating the precipitate by centrifugation and washing it with ethanol three times, it was dispersed into $10^{-3}$ m³ (1 liter) of distilled water. After centrifugal filtration and several washings, fine spherical particles of $10^{-6}$ to $10^{-5}$ m (1—10 $\mu$m) in size were obtained, of which the surface and interior were completely acylated.

From electromicroscopic observation, the ratio of maximum diameter to the minimum diameter of the particles was found to be in the range of from 1.0:1 to 1.1:1. The particles did not dissolve in an aqueous 5% by weight of acetic acid solution.

## Example 5

Three kinds of de-N-acetylated chitin, each having a degree of de-N-acetylation of 0.5, 0.7 and 0.9, respectively, were treated as in Example 1 except that the time over which acylation is effected is changed. The particles obtained had a maximum diameter to minimum diameter ratio of 1.1:1 to 1.3:1, 1.0:1 to 1.1:1, and 1.0:1 to 1.1:1, respectively. By changing the reaction time for acylation, spherically shaped particles were obtained the surface layer of which comprises the insoluble acylated product and the interior of which comprises soluble de-N-acylated chitin. This is shown in the accompanying drawing, in which the sole Figure represents the relationship between the ion-exchange capacity of the particles and the acylation reaction time. The ion-exchange capacity was determined by a batch method.

From the Figure, it can be appreciated that ion-exchange capacity is decreased as the reaction time increases. As the ion-exchange capacity depends on the amount of free amino groups in the particles, the gradual decrease of the ion-exchange capacity corresponds to a gradual increase in the acylation of the amino groups. In other words, acylation and insolubilization of the particles proceed from the surface of the particles while leaving the amino group-containing de-N-acetylated chitin in the interior of the particles. In this manner, the present invention can provide spherical particles, at least the surface layer of which is acylated and insolubilised and which have an optional ion-exchange capacity depending on the acylation reaction time.

The following experiments have also established that particles according to the present invention can have an outer surface comprising the insoluble acylated product and an interior comprising the soluble chitin derivative.

Particles obtained by acylating the above-mentioned de-N-acetylated chitin with a degree of de-N-acetylation of 0.9 for 15 minutes were immersed in an aqueous 5% by weight solution of acetic acid. The particles maintained their spherical shape in the acetic acid solution. After filtration, the filtrate was neutralized with 1 N sodium hydroxide solution. No precipitate appeared but a clear solution remained. This showns that the particles were insoluble in the dilute acid solution and that the soluble de-N-acetylated chitin contained inside the particles was not dissolved in the acidic solution through the acylated and insolubilized surface membrane of the particles.

The particles were crushed in another aqueous 5% by weight solution of acetic acid to break the insoluble surface membrane. By centrifugal filtration, the insoluble pieces of the membrane and the filtrate were separated. Infrared spectroscopic investigations revealed that the insoluble pieces did not have 1500 to 1530 cm$^{-1}$ absorption band of an amino group. This shows that the insoluble membrane was completely acylated and that no amino group was present in the surface of the particles. The filtrate was neutralized with 1 N sodium hydroxide solution to form a cloudy solution. This solution was centrifuged. The precipitate obtained was washed and freeze-dried. The freeze-dried product was observed by the infrared inspection to have an amino absorption band at 1,500 to 1,530 cm$^{-1}$. This shows that the precipitate is the de-N-acetylated chitin originally present in the interior of the particles.

## Example 6

The spherical gel-like particles obtained in Example 1 were dehydrated by centrifugation until the water content of the material became 2 kg per kg (2 g per g) of the dry weight of the

particles. These semi-dried particles was put into 20,000 times their weight of an aqueous 45% sodium hydroxide solution at a temperature of 10°C. The mixture was mixed for 3 hours. The thus-treated particles were deprived of excess alkali until the alkali content of the particles became three times by weight of the dry weight of the particles. After maintaining the particles at a temperaure of 0°C for 2 hours, they were frozen at −25°C for one hour.

An amount of epibromohydrin corresponding to twice the molar amount of the dried particles pre-treated with alkali was dissolved in an amount of isopropyl alcohol corresponding to 50 times the weight of the dried particles pre-treated with alkali. This solution was kept at a temperature of 0 to 5°C and the frozen particles pre-treated with alkali were added to it to effect cross-linking. The reaction was carried out for 5 hours under agitation at from 0 to 5°C and for a further 5 hours at 20°C. Then, the reaction mixture was filtered. After washing the precipitate with ethanol, the precipitate was dispersed in distilled water. The dispersion was then neutralized with a 1 N-hydrochloric acid solution while cooling.

The neutralized dispersion was filtered. The residue was washed with distilled water. It was found that the cross-linked particles maintained their original spherical shape. Infrared absorption spectroscopy established that they had not been de-N-acetylated. The degree of cross-linking of the particles was found to be 0.15 per pyranose ring from elementary analytical data.

**Claims**

1. A process for producing a spherically shaped material having an outer surface layer which comprises an acylated de-N-acetylated chitin or a salt thereof, which process comprises contacting an aqueous solution of a de-N-acetylated chitin or a salt thereof with a liquid containing an organic acid anhydride and a suspending agent in such a manner as to produce the material in the desired shape.

2. A process according to claim 1, wherein the organic acid anhydride has from one to twenty carbon atoms, or a mixture of such anhydrides is used.

3. A process according to claim 1 or 2, wherein the organic acid anhydride is present in an amount of from 5 to 20 equivalents per equivalent of amino groups of the de-N-acetylated chitin.

4. A process according to any one of the preceding claims, wherein the suspending agent is a polyoxyethylene sorbitan monoester or a sorbitan monoester and is present in an amount of from 0.001 to 10% by weight of the amount of the aqueous solution of the de-N-acetylated chitin.

5. A process according to any one of the preceding claims, wherein the aqueous solution of the de-N-acetylated chitin contains a suspending agent.

6. A process according to any one of the preceding claims, wherein said liquid contains an organic acid.

7. A process according to any one of the preceding claims, wherein the organic acid anhydride is dissolved in an organic solvent inert to the reactants and the solvent is added to the reaction system in an amount of from 10 to 500 times by weight of the amount of the de-N-acetylated chitin.

8. A process according to any one of the preceding claims, wherein the aqueous solution of de-N-acetylated chitin is added dropwise to said liquid and is dispersed therein to obtain the spherically shaped material.

9. A process of any one of the preceding claims, wherein the aqueous solution of the de-N-acetylated chitin contains a viscosity-reducing agent.

10. A process according to any one of the preceding claims, wherein the de-N-acetylated chitin or salt thereof to be acylated is represented by the following averaged general formula:

$$[C_6H_9O_4 \cdot (NH_2)_x \cdot (NHCOCH_3)_y]_n$$

wherein x is from 0.5 to 1.0 and $y = 1.0 - x$, or is a salt thereof.

11. A process according to any one of the preceding claims, which is effected such that the interior of the spherically shaped material which is produced comprises de-N-acetylated chitin, or a salt thereof, which has not been acylated.

12. A process according to claim 11 wherein the de-N-acylated chitin in the interior of the material is cross-linked, the degree of cross-linking being up to 0.3 per pyranose ring.

13. A process according to any one of claims 1 to 10, which is effected such that the interior of the spherically shaped material which is produced consists entirely or almost entirely of the acylated de-N-acetylated chitin.

14. A process according to any one of the preceding claims, wherein the degree of de-N-acetylation of the de-N-acetylated chitin is from 0.5 to 0.9.

15. A process according to any one of the preceding claims, which is effected to produce particles having a diameter of from $10^{-7}$ to $5 \times 10^{-3}$ m (0.1 to 5,000 $\mu$m) and a maximum to minimum diameter ratio of from 1.0:1 to 1.3:1 in the wet state.

16. A process according to any one of the preceding claims, wherein the acylated de-N-acetylated chitin is cross-linked.

17. A process according to claim 16, wherein the degree of cross-linking is up to 0.3 per pyranose ring.

18. A process according to any one of the preceding claims, wherein the salt is an acetate or a hydrochloride.

19. Use of a spherically shaped material which has been prepared by a process as claimed in any one of the preceding claims as an adsorbent in a chromatography column, as base material on which an enzyme, antibody or antigen is immobilised, in dialysis, in ultrafiltration, in blood perfusion or for a pad for surgical injuries.

**Revendications**

1. Procédé de production d'un matériau de forme sphérique ayant une couche de surface externe qui comprend une chitine de-N-acétylée acylée ou un sel de celle-ci, lequel procédé comprend la mise en contact d'une solution aqueuse de chitine de-N-acétylée ou un sel de celle-ci avec un liquide contenant un anhydride d'acide organique et un agent de suspension de façon à produire de matériau sous la forme désirée.

2. Procédé selon la revendication 1, caractérisé en ce que l'anhydride de l'acide organique a de 1 à 20 atomes de carbone, ou bien un mélange de tels anhydrides est utilisé.

3. Procédé selon la revendication 1 ou 2, caractérisé en ce que l'anhydride d'acide organique est présent en quantité de 5 à 20 équivalents par équivalent de groupes amino de chitine de-N-acétylée.

4. Procédé selon l'une quelconque des revendications précédentes, caractérisé en ce que l'agent de suspension est un mono-ester de sorbitane polyoxyéthylène ou un monoester de sorbitane et est présent en quantité de 0,001 à 10% en poids de la quantité de solution aqueuse de chitine de-N-acétylée.

5. Procédé selon l'une quelconque des revendications précédentes, caractérisé en ce que la solution aqueuse de chitine de-N-acétylée contient un agent de suspension.

6. Procédé selon l'une quelconque des revendications précédentes, caractérisé en ce que le liquide contient un acide orgainique.

7. Procédé selon l'une quelonque des revendications précédentes, caractérisé en ce que l'anhydride de l'acide organique est dissous dans un solvant organique inerte aux réactifs et le solvent est ajouté ou système réactionnel en quantité de 10 à 500 fois en poids la quantité de chitine de-N-acétylée.

8. Procédé selon l'une quelconque des revendications précédentes, caractérisé en ce que la solution aqueuse de chitine de-N-acétylée est ajoutée goutte à goutte audit liquide et dispersée pour obtenir le matériau de forme sphérique.

9. Procédé selon l'une quelconque des revendications précédentes, caractérisé en ce que la solution aqueuse de chitine de-N-acétylée contient un agent réduisant la viscosité.

10. Procédé selon l'une quelconque des revendications précédentes, caractérisé en ce que la chitine de-N-acétylée ou un sel de celle-ci à acyler est représenté par la formule générale suivante:

$$[C_6H_9O_4\cdot(NH_2)_x\cdot(NHCOCH_3)_y]_n$$

dans laquelle x est de 0,5 à 1,0 et Y = 1,0 − x ou est un sel de celle-ci.

11. Procédé selon l'une quelconque des revendications précédentes, caractérisé en ce qu'il est effectué de sorte que l'intérieur du matériau de forme sphérique qui est produit, comprend de la chitine de-N-acétylée ou un sel de celle-ci, qui n'a pas été acylée.

12. Procédé selon la revendication 11, caractérisé en ce que la chitine de-N-acétylée dans l'intérieur du matériau est réticulée, le degré de réticulation allant jusqu'à 0,3 par noyau de pyranose.

13. Procédé selon l'une quelconque des revendications 1 à 10, qui est effectué de sorte que l'intérieur de matériau de forme sphérique qui est obtenu, consiste entièrement ou presque entièrement en chitine de-N-acétylée acylée.

14. Procédé selon l'une quelconquel des revendications précédentes, caractérisé en ce que le degré de de-N-acétylation de chitine de-N-acétylée est de 0,5 à 0,9.

15. Procédé selon l'une quelconque des revendications précédentes, qui est effectué pour fournir des particules ayant un diamètre de $10^{-7}$ à $5 \times 10^{-3}$ m (0,1 à 5 000 $\mu$m) et un rapport de diamètre maximum à minimum de 1,0/1 à 1,3/1, à l'état humide.

16. Procédé sel on l'une quelconque des revendications précédentes, caractérisé en ce que la chitine de-N-acétylée acylée est réticulée.

17. Procédé selon la revendication 16, caractérisé en ce que le degré de réticulation va jusqu'à 0,3 par noyau de pyranose.

18. Procédé selon l'une quelconque des revendications précédentes, caractérisé en ce que le sel est un acétate ou un chlorhydrate.

19. Application d'un matériau de forme sphérique qui a été préparé selon le procédé défini dans l'une quelconque des revendications précédentes, comme adsorbant dans une colonne de chromatographie comme matériau de base sur lequel un enzyme, un anticorps ou un antigène est immobilisé, en dialyse, en ultrafiltration, en perfusion sanguine ou comme tampon pour les blessures chirurgicales.

**Patentansprüche**

1. Verfahren zur Herstellung eines sphärisch geformten Materials mit einer äußeren Oberflächenschicht, die ein acyliertes N-desacetyliertes Chitin oder ein Salz davon enthält, dadurch gekennzeichnet, daß eine wäßrige Lösung eines N-desacetylierten Chitins oder eines Salzes davon mit einer ein Anhydrid einer organischen Säure und ein Suspendiermittel enthaltenden Flüssigkeit derart in Kontakt gebracht wird, daß das Material in der gewünschten Form gebildet wird.

2. Verfahren nach Anspruch 1, dadurch gekennzeichnet, daß das Anhydrid der organischen Säure 1 bis 20 Kohlenstoffatome aufweist oder daß eine Mischung solcher Anhydride verwendet wird.

3. Verfahren nach den Ansprüchen 1 oder 2, dadurch gekennzeichnet, daß das Anhydrid der organischen Säure in einer Menge von 5 bis 20 Äquivalenten pro Äquivalent Aminogruppen des N-desacetylierten Chitins vorhanden ist.

4. Verfahren nach einem der vorhergehenden Ansprüche, dadurch gekennzeichnet, daß das Suspendiermittel ein Polyoxyethylsorbitanmonoester oder ein Sorbitanmonoester ist und in einer Menge von 0,001 bis 10 Gew.-%, bezogen auf die Menge der wäßrigen Lösung des N-desacetylierten Chitins, vorhanden ist.

5. Verfahren nach einem der vorhergehenden Ansprüche, dadurch gekennzeichnet, daß die wäßrige Lösung des N-desacetylierten Chitins ein Suspendiermittle enthält.

6. Verfahren nach einem der vorhergehenden Ansprüche, dadurch gekennzeichnet, daß die Flüssigkeit eine organische Säure enthält.

7. Verfahren nach einem der vorhergehenden Ansprüche, dadurch gekennzeichnet, daß das Anhydrid der organischen Säure in einem gegenüber des Reaktionsteilnehmern inerten organischen Lösungsmittel gelöst ist und das Lösungsmittel dem Reaktionssystem in einer Menge zugesetzt wird, die dem 10- bis 500-fachen des Gewichts der Menge des N-desacetylierten Chitins entspricht.

8. Verfahren nach einem der vorhergehenden Ansprüche, dadurch gekennzeichnet, daß die wäßrige Lösung des N-desacetylierten Chitins zur Bildung des sphärisch geformten Materials tropfenweise zu der Flüssigkeit zugegeben und darin dipergiert wird.

9. Verfahren nach einem der vorhergehenden Ansprüche, dadurch gekennzeichnet, daß die wäßrige Lösung des N-desacetylierten Chitins ein die Viskosität verminderndes Mittel enthält.

10. Verfahren nach einem der vorhergehenden Ansprüche, dadurch gekennzeichnet, daß das zu acylierende N-desacetylierte Chitin oder dessen Salz der folgenden durchschnittlichen allgemeinen Formel

$$[C_6H_9O_4\cdot(NH_2)_x\cdot(NHCOCH_3)_y]_n$$

in der x 0,5 bis 1,0 und y 1,0 — x bedeuten, oder einem Salz davon entspricht.

11. Verfahren nach einem der vorhergehenden Ansprüche, dadurch gekennzeichnet, daß es in der Weise durchgeführt wird, daß das Innere des gebildeten sphärisch geformten Materials nicht-acyliertes N-desacetyliertes Chitin oder ein Salz davon enthält.

12. Verfahren nach Anspruch 11, dadurch gekennzeichnet, daß das im Inneren des Materials vorliegende N-desdacetylierte Chitin vernetzt ist, wobei der Vernetzungsgrad bis zu 0,3 pro Pyranosering beträgt.

13. Verfahren nach einem der Ansprüche 1 bis 10, dadurch gekennzeichnet, daß es in der Weise durchgeführt wird, daß das Innere des gebildeten sphärisch geformten Materials ausschließlich oder fast ausschließlich aus dem acylierten N-desactylierten Chitin besteht.

14. Verfahren nach einem der vorhergehenden Ansprüche, dadurch gekennzeichnet, daß der Grad der N-Desacetylierung des N-desacetylierten Chitins 0,5 bis 0,9 beträgt.

15. Verfahren nach einem der vorhergehenden Ansprüche, dadurch gekennzeichnet, daß es nin der Weise durchgeführt wird, daß Teilchen gebildet werden, diein feuchtem Zustand einen Durchmesser von $10^{-7}$ bis $5 \times 10^{-3}$ m (0,1 bis 5000 $\mu$m) und ein Verhältnis von maximalem Durchmesser zu minimalem Durchmesser von 1,0:1 bis 1,3:1 aufweisen.

16. Verfahren nach einem der vorhergehenden Ansprüche, dadurch gekennzeichnet, daß das acylierte N-desacetylierte Chitin vernetzt wird.

17. Verfahren nach Anspruch 16, dadurch gekennzeichnet, daß der Vernetzungsgrad bis zu 0,3 pro Pyranosering beträgt.

18. Verfahren nach einem der vorhergehenden Ansprüche, dadurch gekennzeichnet, daß das Salz ein Acetat oder ein Hydrochlorid ist.

19. Verwendung eines nach dem Verfahren nach einem der vorhergehenden Ansprüche hergestellten sphärisch geformten Materials als Adsorbens in Chromatographiesäulen, als Grundmaterial, auf dem Enzym, ein Antikörper oder ein Antigen immobilisert wird, bei der Dialyse, bei der Ultrafitration, bei der Blutperfusion oder als Kompresse für Operationswunden.

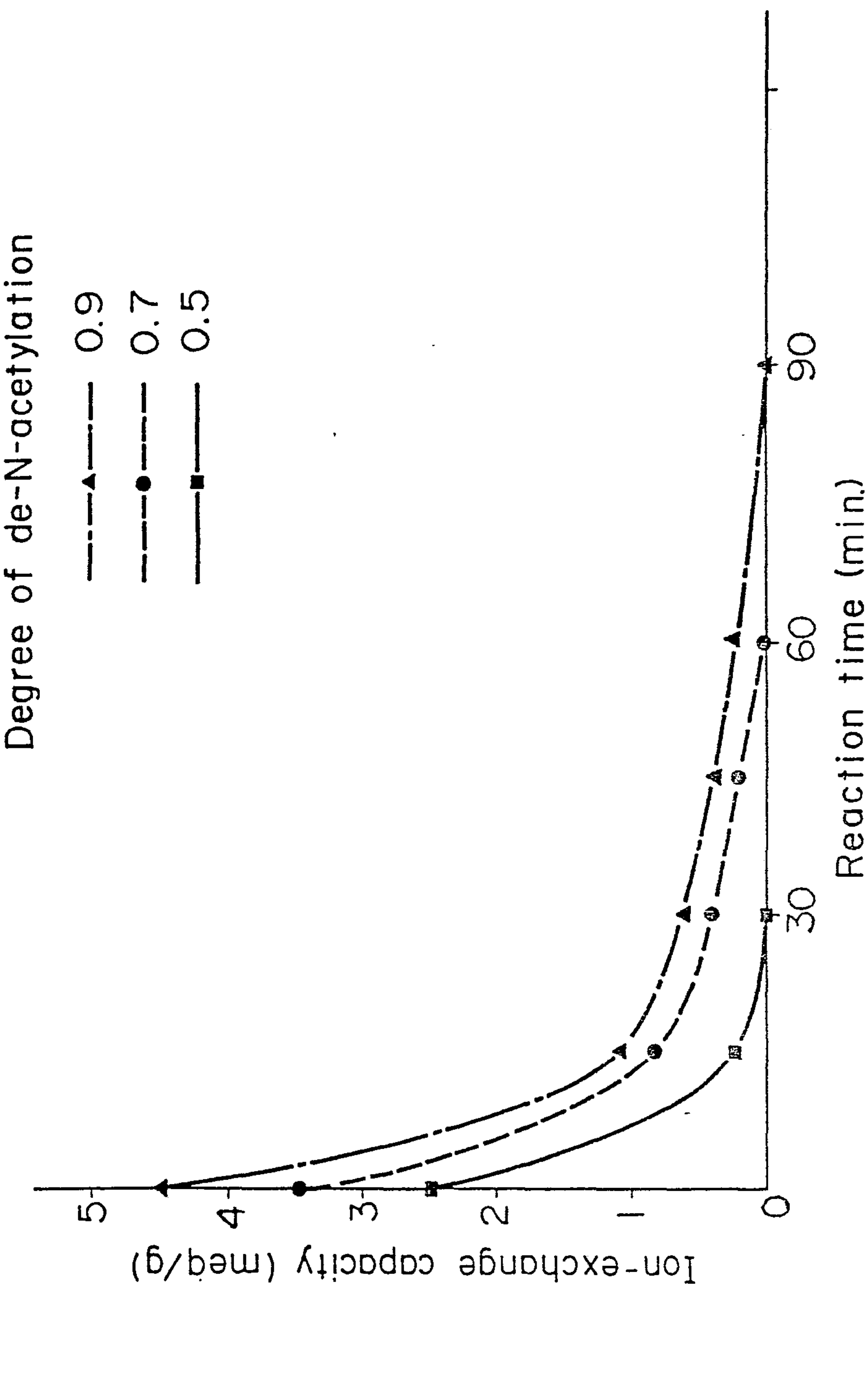
Degree of de-N-acetylation
0.9
0.7
0.5
Ion-exchange capacity (meq/g)
0
1
2
3
4
5
Reaction time (min.)
30
60
90